# EUROPEAN PATENT SPECIFICATION

(11) **EP 1 319 416 B1**
(45) Date of publication and mention of the grant of the patent: **03.11.2004**
(21) Application number: 01129570.6
(22) Date of filing: 12.12.2001
(51) Int. Cl.: A61L 31/16, A61L 31/08

(54) **Porous metallic stent with a ceramic coating**
Poröser Metallstent mit keramischer Beschichtung
Stent métallique poreux avec un revêtement céramique

(43) Date of publication of application: 18.06.2003
(73) Proprietor: Hehrlein, Christoph, Dr., 79104 Freiburg (DE)
(72) Inventor: Hehrlein, Christoph, D-79104 Freiburg (DE); Kovacs, Adalbert, D-69214 Eppelheim (DE); Wolf, Gerhard Karl, D-69120 Heidelberg (DE)
(74) Representative: KOHLER SCHMID + PARTNER

(56) References cited:
- EP-A- 0 875 217
- EP-A- 0 895 761
- WO-A-01/17577
- US-A- 5 649 951

## Description

### Field of the Invention

This invention relates to porous metallic stents and methods for producing such stents being capable to incorporate and elute pharmacologically active agents. The purpose of these stents is to prevent restenosis after angioplasty.

### Background of the Invention

A percutaneous transluminal angioplasty (PTA) of blood vessels and more specifically the angioplasty of coronary arteries (PTCA) is a very popular method to eliminate narrowing or stenosis that obstruct blood flow to human organs. Endovascular stents are used as scaffolding devices to prevent abrupt closure of arteries undergoing angioplasty. Stents are also capable of reducing restenosis rates compared with conventional balloon angioplasty. However, restenosis after stent-implantation remains a problem with rates of 20-30% in coronary arteries. Restenosis is the result of excessive damage to the vessel wall during stent placement. Excessive neointima formation within the stents is known to be the major component of restenosis. Restenosis rates after stenting appear to be influenced by the stent design as shown by Rogers et al. (Circulation 1995; 91:2995-3001), and the stent material. Holmes et al (J Am Coll Cardiol 1994; 24:525-531) for instance showed that certain polymers used for the production of drug eluting stents coatings do not decrease but even increase neointima formation. In WO 90/13332 and WO 91/12779, stents have been disclosed that are coated with anti-thrombotic and anti-inflammatory drugs to reduce restenosis rates. In WO 99/00071 another method of stent coating is disclosed by which a DNA coated stent is proposed to reduce restenosis rates. One of the promising drugs that appear to effectively reduce restenosis rates is the substance rapamycin (sirolimus). Sousa et al (Circulation 2001; 103:192-195) showed that a rapamycin-eluting stent reduces neointima formation within the stent, and substantially lower restenosis rates in patients. However, alternative cell-cycle inhibitors may also be effective drugs eluting from stents to prevent restenosis. For instance, US 6200985 disclosed rapamycin derivates that are lowering restenosis rates after angioplasty administered orally. Calcium channel blockers and angiotensin converting enzyme inhibitors did not reduce restenosis rates when administered systemically. However, high concentrations of these drugs acting locally at the site of angioplasty by eluting from a stent may be alternatives to rapamycin eluting stents. Recently, Brown et al (NEJM 2001; 345: 1583) have shown the combination of simvastatin and niacin reduce cardiovascular events when administered orally. In additions, several antibiotics are considered for prevention of progression of atherosclerosis in coronary artery disease.

US 5 649 951 A discloses a metallic stent with a first coating of ceramic zirconium oxide and a second coating of an active agent. The ceramic coating is applied to obtain corrosion resistance and blood compatilibity.

EP 0 875 217 A discloses a porous metal stent formed of a plurality of wires to improve the strength of the stent system. The pores are additionally filled with a therapeutic agent.

### Summary of the Invention

Porous surfaces have long been known a potential reservoir for liquid drugs. The invention describes porous metallic stents capable of incorporating and eluting drugs. Methods are disclosed for producing a porous metallic stent via processes of pitting. Furthermore, methods are disclosed for producing a polymer coating or ceramic coating of the porous metallic stent via techniques of spotted film deposition. The invention described herein discloses a distinct surface modification of a metallic stent to allow a sustained drug release at various intensities from the stent. However, highly porous metallic stent surfaces may contribute to adhesion of platelets and fibrin, for instance after complete elution of a incorporated drug , and may have disadvantages in the preservation of vessel patency. In addition, highly porous metallic stents need to be stabilised with coatings to reduce the risk of cracks and fissures, or the breaking of stent struts. In the invention described herein, methods of making a porous metallic stent are followed by processes to stabilise the porous stent platform to maintain stent stability, flexibility and ease of deployment. One method described herein is designed to improve biocompatibility of a porous stent by applying a ceramic surface coating onto the porous stent surface. Ceramic coating for surgical instruments have been disclosed in WO98/45225 to form a hardened surface with improve gripping ability of the instruments. WO 00/43572 discloses non-porous ceramic layers for the protection of metallic surfaces. Polymer coating s of metallic stents have become known as reservoirs for pharmacologically active agents. According to Sousa et al (Circulation 2001), drugs eluting from endovascular stents covered with thin polymer films appear to be useful instruments to prevent restenosis after angioplasty in coronary arteries. The methods described herein differ from previous ones by disclosing ways of polymer spot coating of a metallic stent surface. In addition to a ceramic stent coating of highly porous stent, this invention consists of the polymer coating of the pores leaving the remainder of the metallic surface non-coated. The methods disclosed herein provide a cost effective means of producing endovascular stents with the capacity of incorporating high quantities of drugs for sustained drug release. The methods described herein included an introduction of polymers into the microholes of a porous stent. The pores are filled with one or multiple polymers incorporating pharmacologically active agents that are released over time.

Galvele JR (In: treatise on material science and technology,vol.23, ed. Scully JC, Academic Press 1983, London, pp1-53) described a process of electrochemically induced pitting of metals in a textbook of material science. Jessensky et al (Appl Phys Let 1998; 72: 1173) reported on electrochemically induced pore arrays in aluminium foils. One method disclosed herein used to produce a porous metallic stent consists of a controlled pitting process using aqueous salt solutions. The solutions may create holes in the surface of a metallic stent at sizes between 500 nm to 50 µm. A variation of the incubation period of the stent with the solution modifies the size of the pores. The variation in fluid characteristics of the solution composition enables the production of holes with different configuration (i.e. more or less shallow, ragged or smoothly edged etc). Highly porous structures enable the intake of great amounts of drugs. The eluting capacity of a pharmacologically active agent incorporated into a stent depends on factors binding the agent to the stent. For instance, polymers attached to a metallic stent may prolong the release kinetics of the pharmacologically active agent. A stent may be coated in part with a polymer, and remains partly uncoated to increase the release kinetics around the uncoated part of the stent. Thus, certain areas of the stents may release the agent faster than others depending on the local coating characteristics. However, the mechanical properties of the stent are also affected by increased porosity. In accordance with the present invention, stent made porous by pitting are further treated with a non-porous or nano-porous films of ceramic material such as titanium dioxide or other ceramics to increase stent stability. Examples of biocompatible ceramic coatings include but are not limited to TiO₂, ZrO₂ or Al₂O₃. Wang et al (Surface Coatings Technol 2000; 128-129: 36) for instance reported that titanium oxide films improve the blood biocompatibility of heart valves.

Example 1: Electrochemically induced pitting under controlled conditions provides a suitable method for porous surface of various metallic stents. Metallic stents made from stainless steel, tantalum, or nitinol are suitable. The electrolyte composition, the applied potential, and the reaction time are important factors that modulate the degree and the morphology of pitting. Stents made from stainless steel 316L for instance are used as working electrodes and are connected with a platinum electrode. The platinum electrode is kept in a slightly acidic NaCI solution at room temperature. A potential of 100 mV is applied, and increased in steps of 0.1 mV/s until 300 mV is reached. By varying the reaction time, different pore sizes can be obtained. Thereafter, the stent is removed, rinsed with distilled water and dried. Prior to electrochemically induced pitting, the metallic stent may be wrapped into microporous foil, which is removed after the pitting process.

Example 2: Metallic stents made porous by the pitting process described in example one are placed into a vacuum chamber. Ceramic material such as titanium dioxide evaporates, and is deposited onto the stent surface by means of argon ion assistance. The energy of the ions may range from 1 to 30 keV. The i/A-ratio (amount of arriving ions/amount of arriving atoms) may vary from 0.01 to 0.1. The thickness of the titanium dioxide layer is controlled to be in the range of 0.5 - 1 microns. Thereafter, the stent is dipped into a liquid solution of a pharmaceutical agent for stent-based drug release. The microfoil may be removed either before or after the film deposition resulting in entirely coated stents or partly coated ones, depending on the location of the foil.

Example 3: Metallic stents made porous by the pitting process described in example one are dipped into a liquid solvent of a polymer material. After the solvent is removed by evaporation, the polymer outside the holes will be detached chemically. The chemical detachment process for the polymer is performed by using other suitable solvents such as CHCl₃. Alternatively, a micro-foil is applied to the stent surface. By these chemical or mechanical processes, the polymer remain inside the pores and is not present on the surface of the stent. The polymer inside the pores stabilises the structure of the stent. Furthermore, the polymer inside the pores of the metallic stent is able to uptake and store liquid solutions. The porous stent is dipped into a liquid solution of a drug or a pharmaceutical agent. The polymer filled pores serve as a reservoir for the pharmacologically active agent and allow sustained release of the agent.

### Description of Drawings

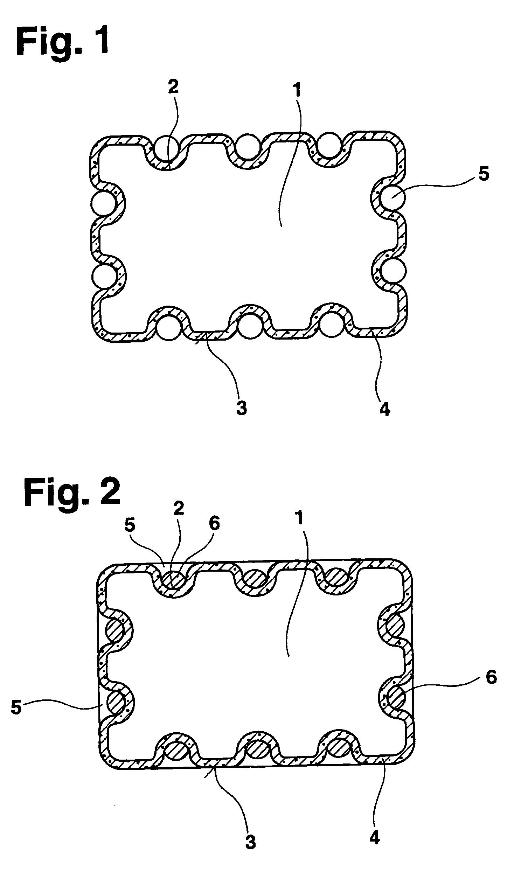

Figure 1 shows a cross-sectional view of a porous metallic stent strut 1 with a ceramic coating. The pores 2 of the stent surface 3 are coated with a thin ceramic layer 4. The metallic part of the stent 1 is entirely covered with the ceramic thin film layer 4. A pharmacologically active agent 5 is present in the coated pores 2 of the stent 1.

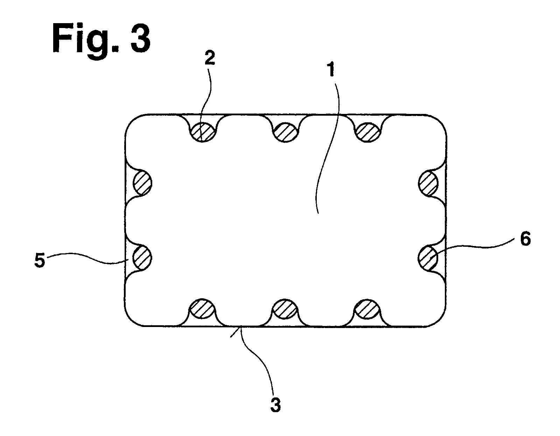

Figure 2 shows a cross-sectional view of a porous metallic stent strut 1 with a ceramic coating. The pores 2 of the stent surface 3 are coated with a thin ceramic layer 4. The metallic part of the stent 1 is entirely covered with the ceramic thin film layer 4. The coated pores 2 are filled with a polymer material 6 located on the base of the pores 2 of the stent 1. The polymer material 6 is loaded with a pharmacologically active agent 5.
Figure 3 shows a cross-sectional view of a porous metallic stent strut 1 with the pores 2 in the stent surface 3. The pores 2 are filled with a polymer material 6 located on the base of the pores 2 of the stent 1. The polymer material 6 is loaded with a pharmacologically active agent 5, The surface 3 of the stent strut 1 is not covered with the polymer material 6.

The invention described herein consists of a porous metallic stent and methods for producing such a stent using the same to prevent restenosis after angioplasty or the progression of atherosclerosis. The pores of the metallic stent are created by electrochemically induced pitting. Additional stent coatings are applied to improve stability of the stent, and to modify the release kinetics of a pharmacologically active agent or drug eluting from the stent.

## Claims

1. A porous metallic stent (1) coated with a ceramic layer (4) and containing a pharmacologically active agent (5), wherein the pores (2) of the stent (1) have the capacity to incorporate and elute pharmacologically active agents (5).

2. The porous metallic stent of Claim 1, wherein the ends of the stent (1) are porous and the middle of the stent (1) is non-porous.

3. The porous metallic stent of claim 1 or 2, wherein the ceramic layer (4) is non-porous.

4. The porous metallic stent of claim 3, wherein a polymer material (6) is located inside the pores (2) of the stent (1).

5. The porous metallic stent of claim 1 or 2, wherein the porous metallic stent (1) is micro-porous and the ceramic layer (4) is nano-porous and wherein the pharmacologically active agent (5) is located in the micro-pores (2) of the porous metallic stent (1) and in the nano-pores of the ceramic layer (4).

6. The porous metallic stent of claim 5, wherein a polymer material (6) is located inside the micro-pores (2) of the porous metallic stent (1).

7. The porous metallic stent of any one of the preceding claims, wherein the ceramic layer (4) consists of either titaniumdioxide(TiO₂), zirconiumdioxide(ZrO₂) or aluminiumoxide (Al₂O₃).

8. A porous metallic stent (1) containing a pharmacologically active agent (5) and a polymer coating (6) of the pores (2) of the stent (1) wherein the pores (2) of the stent (1) have the capacity to incorporate and elute pharmacologically active agents (5).

9. The porous metallic stent of claim 8, wherein the pores (2) in the stent middle are filled with the polymer material (6) and wherein the pores at the stent ends are not filled with the polymer material (6).

10. The porous metallic stent of any one of the preceding claims, wherein the pharmacologically active agent (5) incorporated and eluting from the stent (1) is one of the group of calcium channel blockers (such as verapamil), one of the group of angiotensin converting enzyme inhibitors (such as captopril), one of the group of statins (such as simvastatin), one of the group of immunosuppressants (such as 32 deoxo-rapamycin), or one of the group of antibiotics (such as erythromycin).

11. The porous metallic stent of any one of the preceding claims, wherein the pores (2) of the stent (1) are obtainable by pitting of the metallic stent (1) and wherein pitting is electrochemically induced in an inorganic salt solution applying a pitting potential and different reaction times.

12. The porous metallic stent of Claim 11, wherein the porous stent (1) is further coated by an argon beam assisted film deposition of ceramic layer (4).

13. The porous metallic stent of Claim 11 or 12, wherein the pores (2) of the stent (1) are impregnated with biostable polymer material (6) such as polyethylene, polyethylene oxide, polyethylene terephtalate, ethylene vinyl acetate or silicone.

14. The porous metallic stent of any one of Claims 11 - 13, wherein the pores (2) of the stent (1) are impregnated with biodegradable polymer material (6) such as poly ε-caprolactone (PCL), poly-D,L-lactic acid (DL-PLA), poly-lactide-co-glycolide, poly-β-hydroxybutyrate (PHB), poly-p-dioxanone (PDS), polyorthoester, cyanoacrylates, aliphatic polycarbonates, polyalkylene oxalates, polyiminocarbonates, polyphophazenes.

## Patentansprüche

1. Poröser, metallischer Stent (1), der mit einer keramischen Schicht (4) beschichtet ist und einen pharmakologisch aktiven Wirkstoff (5) aufweist, wobei die Poren (2) des Stents (1) die Fähigkeit haben, pharmakologisch aktive Wirkstoffe (5) aufzunehmen und zu eluieren.

2. Poröser, metallischer Stent nach Anspruch 1, wobei die Enden des Stents (1) porös sind und die Mitte des Stents (1) nicht-porös ist.

3. Poröser, metallischer Stent nach Anspruch 1 oder 2, wobei die keramische Schicht (4) nicht-porös ist.

4. Poröser, metallischer Stent nach Anspruch 3, wobei ein Polymermaterial (6) in den Poren (2) des Stents (1) angeordnet ist.

5. Poröser, metallischer Stent nach Anspruch 1 oder 2, wobei der poröse, metallische Stent (1) mikroporös ist und die keramische Schicht (4) nanoporös ist und wobei der pharmakologisch aktive Wirkstoff (5) in den Mikroporen (2) des porösen, metallischen Stents (1) und in den Nanoporen der keramischen Schicht (4) angeordnet ist.

6. Poröser, metallischer Stent nach Anspruch 5, wobei ein Polymermaterial (6) in den Mikroporen (2) des porösen metallischen Stents (1) angeordnet ist.

7. Poröser, metallischer Stent nach einem der vorhergehenden Ansprüche, wobei die keramische Schicht (4) entweder aus Titandioxid (TiO₂), Zirkondioxid (ZrO₂) oder Aluminiumoxid (Al₂O₃) besteht.

8. Poröser, metallischer Stent (1), der einen pharmakologisch aktiven Wirkstoff (5) und eine Polymerbeschichtung (6) der Poren (2) des Stents (1) enthält, wobei die Poren (2) des Stents (1) die Fähigkeit haben, pharmakologisch aktive Wirkstoffe (5) aufzunehmen und zu eluieren.

9. Poröser, metallischer Stent nach Anspruch 8, wobei die Poren (2) in der Stentmitte mit dem Polymermaterial (6) gefüllt sind und wobei die Poren an den Stentenden nicht mit dem Polymermaterial (6) gefüllt sind.

10. Poröser, metallischer Stent nach einem der vorhergehenden Ansprüche, wobei der pharmakologisch aktive Wirkstoff (5), der in dem Stent (1) aufgenommen ist und daraus eluiert wird, einer aus der Gruppe von Calciumkanalblockern (wie Verapamil), einer aus der Gruppe von Angiotensin umwandelnden Enzymhemmstoffen (wie Captopril), einer aus der Gruppe von Statinen (wie Simvastatin), einer aus der Gruppe von Immunosuppressiva (wie 32 Deoxo-Rapamycin) oder einer aus der Gruppe von Antibiotika (wie Erythromycin) ist.

11. Poröser, metallischer Stent nach einem der vorhergehenden Ansprüche, wobei die Poren (2) des Stents (1) durch Grübchenkorrosion (Pitting) des metallischen Stents (1) erhalten werden können und wobei die Grübchenkorrosion elektrochemisch in einer anorganischen Salzlösung induziert wird durch Anlegen eines Grübchenkorrosionspotentials und Verwendung unterschiedlicher Reaktionszeiten.

12. Poröser, metallischer Stent nach Anspruch 11, wobei der poröse Stent (1) weiterhin durch eine Filmablagerung einer keramischen Schicht (4) mit Unterstützung eines Argonstrahls beschichtet ist.

13. Poröser, metallischer Stent nach Anspruch 11 oder 12, wobei die Poren (2) des Stents (1) mit biologisch stabilem Polymermaterial (6), wie Polyethylen, Polyethylenoxid, Polyethylenterephthalat, Ethylenvinylacetat oder Silicon, imprägniert sind.

14. Poröser metallischer Stent nach einem der Ansprüche 11-13, wobei die Poren (2) des Stents (1) mit biologisch abbaubarem Polymermaterial (6), wie Poly-ε-Caprolacton (PCL), Poly-D,L-Milchsäure (DL-PLA), Poly-Lactid-co-Glycolid, Poly-β-Hydroxybutyrat (PHB), Poly-p-Dioxanon (PDS), Polyorthoester, Cyanacrylaten, aliphatischen Polycarbonaten, Polyalkylenoxalaten, Polyiminocarbonaten, Polyphophazenen, imprägniert sind.

## Revendications

1. Stent métallique poreux (1) recouvert d'une couche céramique (4) et contenant une substance pharmacologiquement active (5), dans lequel les pores (2) du stent (1) ont la capacité d'incorporer et d'éluer des substances pharmacologiquement actives (5).

2. Stent métallique poreux (1) selon la revendication 1, dans lequel les extrémités du stent (1) sont poreuses et la partie médiane du stent (1) est non-poreuse.

3. Stent métallique poreux selon la revendication 1 ou 2, dans lequel la couche céramique (4) est non-poreuse.

4. Stent métallique poreux selon la revendication 3, dans lequel un matériau polymère (6) se trouve à l'intérieur des pores (2) du stent (1).

5. Stent métallique poreux selon la revendication 1 ou 2, dans lequel le stent métallique poreux (1) est micro-poreux et la couche céramique (4) est nano-poreuse et dans lequel la substance pharmacologiquement active (5) se trouve à l'intérieur des micro-pores (2) du stent métallique poreux (1) et les nano-pores de la couche de céramique (4).

6. Stent métallique poreux selon la revendication 5, dans lequel un matériau polymère (6) se trouve à l'intérieur des micro-pores (2) du stent métallique poreux (1).

7. Stent métallique poreux selon l'une quelconque des revendications précédentes, dans lequel la couche de céramique (4) se compose à la fois de dioxyde de titane (TiO₂), de dioxyde de zirconium (ZrO₂) ou d'alumine (Al₂O)₃).

8. Stent métallique poreux (1) contenant une substance pharmacologiquement active (5) et un revêtement polymère (6) à l'intérieur des pores (2) du stent (1) dans lequel les pores (2) du stent (1) ont la capacité d'incorporer et d'éluer des substances pharmacologiquement actives (5).

9. Stent métallique poreux selon la revendication 8, dans lequel les pores (2) de la partie médiane du stent sont remplis avec le matériau polymère (6) et dans lequel les pores (2) aux extrémités du stent ne sont pas remplis avec le matériau polymère (6).

10. Stent métallique poreux selon l'une quelconque des revendications précédentes, dans lequel la substance pharmacologiquement active (5) incorporée et éluant du stent (1) est une substance du groupe des inhibiteurs du canal calcique (tel le vérapamil), une substance du groupe des inhibiteurs de l'enzyme de conversion de l'angiotensine (tel le captopril), une substance du groupe de la statine (telle la simvastatine), une substance du groupe des immunosuppresseurs (tel que la 32-deoxorapamycine), ou une substance du groupe des antibiotiques (telle que l'érythromycine).

11. Stent métallique poreux selon l'une quelconque des revendications précédentes, dans lequel les pores (2) du stent (1) peuvent être obtenus par perforation du stent métallique (1) et dans lequel les perforations sont induites électrochimiquement dans une solution de sel inorganique en appliquant un potentiel de perforation et différents temps de réaction.

12. Stent métallique poreux selon la revendication 11, dans lequel le stent poreux (1) est de plus revêtu d'une couche de céramique (4) par dépôt de film assisté par faisceau d'argon.

13. Stent métallique poreux selon la revendication 11 ou 12, dans laquelle les pores (2) du stent (1) est imprégné avec un matériau polymère biostable (6) tel que le polyéthylène, l'oxyde de polyéthylène, le polyéthylène téréphtalate, l'éthylène acétate de vinyle ou le silicone.

14. Stent métallique poreux selon l'une quelconque des revendications 11 à 13, dans lequel les pores (2) du stent (1) sont imprégnés avec un matériau polymère biodégradable (6) tel que le poly ε-caprolactone (PCL), le D,L-poly(acide lactique) (DL-PLA), le poly-lactide-co-glycolide, le poly-β-hydroxybutyrate (PHB), la poly-p-dioxanone (PDS), le polyorthoester, les cyanoacrylates, les polycarbonates aliphatiques, les oxalates de polyalkylène, les polyiminocarbonates, les polyphophazènes.
